(19) Europäisches Patentamt
European Patent Office
Office européen des brevets

(11) **EP 2 230 259 A1**

(12) **EUROPÄISCHE PATENTANMELDUNG**

(43) Veröffentlichungstag:
22.09.2010  Patentblatt 2010/38

(51) Int Cl.:
*C08B 37/14* (2006.01)     *C08G 73/02* (2006.01)

(21) Anmeldenummer: 09450153.3

(22) Anmeldetag: 25.08.2009

(84) Benannte Vertragsstaaten:
AT BE BG CH CY CZ DE DK EE ES FI FR GB GR
HR HU IE IS IT LI LT LU LV MC MK MT NL NO PL
PT RO SE SI SK SM TR
Benannte Erstreckungsstaaten:
AL BA RS

(30) Priorität: 18.03.2009  EP 09450060

(71) Anmelder: Mindinvest Holdings Ltd.
1122 Valleta VLT, MT (MT)

(72) Erfinder: Schmidt, Oskar
1030 Wien (AT)

(74) Vertreter: Schwarz, Albin
Schwarz & Partner
Patentanwälte
Wipplingerstraße 30
1010 Wien (AT)

(54) **Mikrobiozid wirkendes Polymergemisch**

(57)   Mikrobiozid wirkendes Polymergemisch, welches ein erstes Polymer als Matrix aufweist, in welche ein zweites Polymer eingebracht ist, wobei das zweite Polymer ein polymeres Guanidin- oder Biguanidinderivat ist, **dadurch gekennzeichnet, dass** das mikrobiozid wirkende Polymergemisch als Pulver ausgebildet ist.

EP 2 230 259 A1

**Beschreibung**

Die vorliegende Erfindung betrifft ein mikrobiozid wirkendes Polymergemisch, welches ein erstes Polymer als Matrix aufweist, in welche ein zweites Polymer eingebracht ist, welches mikrobiozid wirkt.

Mikrobiozid wirkende Polymere auf der Basis von Guanidinium-Hydrochlorid, insbesondere deren Wirkung gegen *Escherichia coli*-Bakterien sind bereits bekannt (vgl. WO 01/85676). Weiterhin ist bereits bekannt, dass solche Guanidin-Derivate als fungizide Mittel verwendet werden können (vgl. WO 2006/047800). Von besonderer Bedeutung sind die Polymere Akacid®, das Poly-[2-(2-ethoxy)-ethoxyethyl-guanidiniumchlorid], und Akacid plus®, eine 3:1-Mischung aus Poly-(hexamethylenguanidiniumchlorid) und Poly-[2-(2-ethoxy)-ethoxyethyl)-guanidiniumchlorid] (vgl. Antibiotika Monitor, 22. Jahrgang, Heft 1/2/2006, Online-Ausgabe unter http://www.antibiotikamonitor.at/06_12/06_12_inhalt.htm).

Die genannten mikrobizid wirkenden Polymere gehören zur Gruppe der kationischen Antiseptika, welche chemisch sehr unterschiedliche Substanzen umfassen, die aber als gemeinsames Charakteristikum stark basische Gruppen, gebunden an ein ziemlich massives lipophiles Molekül, besitzen. Die wichtigsten Vertreter sind unter den Quarternären Ammoniumverbindungen Benzalkoniumchlorid und Cetrimid, unter den Bisbiguaniden Chlorhexidin und Alexidin und unter den polymerischen Biguaniden Polyhexamethylen-Biguanid (PHMB).

Die kationisch antimikrobiell wirksamen Substanzen werden seit vielen Jahren zur Antisepsis und Desinfektion innerhalb und außerhalb von klinischen Einrichtungen eingesetzt. Aufgrund ihrer eigenen positiv geladenen Moleküle haben die kationisch antimikrobiell wirksamen Substanzen eine hohe Bindungsaffinität an die negativ geladenen Zellwände und Membranen von Bakterien. Durch Störung dieser Angriffspunkte kommt es zunächst zur Herabsetzung der Membranfluidität und zu einer Störung der osmoregulatorischen und physiologischen Zellfunktionen. In weiterer Folge entstehen hydrophile Poren in der Phospholipidmembran, und die Proteinfunktion wird gestört. Das Endresultat ist eine Lyse der Zielzelle. Dieser Membran-schädigende Wirkmechanismus konnte auch für die polymerischen Guanidine gegenüber *Escherichia coli* demonstriert werden.

Aufgrund dieser Eigenschaft werden kationisch antimikrobiell wirksame Substanzen oft als Additiv verschiedensten Materialien zugesetzt, um z.B. ein Wachsen von Bakterien zu verhindern. Sie weisen als kationisch geladene Moleküle jedoch den Nachteil auf, dass sie mit anionischen Bestandteilen, die z.B. in Farben und Lacken, in Beschichtungsmaterialien und in Silikonverfugmassen vorhanden sein können, unerwünschte Wechselwirkungen eingehen, wodurch die mikrobizide Wirkung beseitigt oder zumindest erheblich vermindert wird, oder wodurch es zu unerwünschten Koagulationserscheinungen kommt.

Hier setzt nun die vorliegende Erfindung an, die sich die Aufgabe stellt, den Einsatzbereich der mikrobizid wirkenden polymeren Guanidine und Biguanidine insofern zu erweitern, dass sie eben auch als mikrobizid wirkendes Additiv für Materialen eingesetzt werden können, welche anionische Bestandteile aufweisen.

Diese Aufgabe wird dadurch gelöst, indem die mikrobizid wirkenden polymeren Guanidine oder Biguanidine in ein Polymer eingebracht werden, wonach das Polymergemisch zu einem Pulver vermahlen wird. Das Polymer wirkt als Matrix, aus der die Guanidine bzw. Biguanidine offenbar nicht nennenswert herausgewaschen werden können, weil es nicht zu den erwähnten nachteiligen Effekten kommt. Die mikrobizide Wirkung bleibt aber erhalten.

Die Erfindung betrifft daher ein mikrobiozid wirkendes Polymergemisch, welches ein erstes Polymer als Matrix aufweist, in welche ein zweites Polymer eingebracht ist, wobei das zweite Polymer ein polymeres Guanidin- oder Biguanidinderivat ist, welches Polymergemisch **dadurch gekennzeichnet** ist, dass es als Pulver ausgebildet ist.

Die Erfindung beruht ferner auf der Erkenntnis, dass das Pulver, welches die genannten polymere Guanidine enthält, als Additiv den diversen Materialien zugesetzt werden kann und die mikrobizide Wirkung trotz Anwesenheit von anionischen Komponenten entfaltet. Es kommt auch nicht zu einer Koagulation, was z.B. beim Einsatz in Farben und Lacken von entscheidender Bedeutung ist.

Im Stand der Technik ist ein Acrylat für Kunststoff-Zahnfüllungen bekannt, welches ein polymeres Guanidin enthält (Österreichisches Forschungsinstitut für Chemie und Technik). Dieses Acrylat besitzt eine Plaque-hemmende Wirkung.

Die mittlere Teilchengröße des erfindungsgemäßen Pulvers liegt bevorzugt im Bereich von 10 nm und 1.000 $\mu$m, insbesondere im Bereich von 50 $\mu$m und 200 $\mu$m.

Das polymere Biguanidinderivat kann ein Poly(hexamethylen)biguanid sein.

Das polymere Guanidinderivat ist bevorzugt auf Basis eines Alkylendiamins und/oder eines Oxyalkylendiamins und enthält das Alkylendiamin und das Oxyalkylendiamin im Molverhältnis zwischen 4:1 und 1:4.

Die Aminogruppen des Alkylendiamins und/oder des Oxyalkylendiamins sind bevorzugt endständig.

Zur Herstellung des polymeren Guanidinderivates ist ferner bevorzugt als Alkylendiamin eine Verbindung der allgemeinen Formel

$$NH_2(CH_2)_nNH_2$$

vorgesehen, in welcher n eine ganze Zahl zwischen 2 und 10, insbesondere 6, ist.

Zur Herstellung des polymeren Guanidinderivates ist darüber hinaus bevorzugt als Oxyalkylendiamin eine Verbindung der allgemeinen Formel

$$NH_2[(CH_2)_2O]_n(CH_2)_2NH_2$$

vorgesehen, in welcher n eine ganze Zahl zwischen 2 und 5, insbesondere 2, ist.

Eine weitere bevorzugte Ausführungsform des erfindungsgemäßen Polymergemisch besteht darin, dass als polymeres Guanidinderivat ein Polyoxyalkylen-Guanidin auf Basis von Triethylenglykoldiamin (relative Molekularmasse: 148), von Polyoxypropylendiamin (relative Molekularmasse: 230) und/oder von Polyoxyethylendiamin (relative Molekularmasse: 600) vorgesehen ist.

Als polymeres Guanidinderivat eignet sich Poly-[2-(2-ethoxy-ethoxyethyl)-guanidinium-hydrochlorid] mit mindestens 3 Guanidinresten besonders gut.

Die mittlere Molekularmasse des polymeren Guanidinderivates liegt insbesondere im Bereich 500 bis 3.000.

Das polymere Guanidinderivat kann im ersten Polymer in einer Menge zwischen 0,1 Gew.-% und 20 Gew.-%, bezogen auf die Gesamtmasse, enthalten sein.

Das erste Polymer kann ein Thermoplast, ein Duroplast oder ein Elastomer sein. Als härtende Kunststoffe kommen die Phenoplaste, wie Phenol, Resorcin, Kresol, Xylenol-Harz, sowie die Aminoplaste, wie Melamin- und Harnstoff-Harz, in Frage. Der Wirkstoff (das polymere Guanidin) wird nach der Vorkondensation("Novolak") dem Härter (Hexamethylentetramin)zugesetzt.

Ungesättigte Polyesterharze und ölmodifizierte Polyesterharze werden mit dem Härter vermischt, wobei der Härter vorzugsweise ein Peroxyd ist und der Wirkstoff dem Härter zugesetzt wird.

Epoxyharze werden u.a. mit Polyalkohol-Phenol-Amid oder -Amin gehärtet, wobei der Wirkstoff jeweils dem Härter zugesetzt wird.

Bei Polyurethanharz wird der Wirkstoff der Polyol-Komponente zugesetzt.

Bei Polymethylmethacrylat-Harz wird der Wirkstoff ebenfalls dem Härter zugesetzt.

Die Erfindung ist nicht auf die angeführten Beispiele von härtenden Kunststoffen beschränkt. Bei der Auswahl handelt es sich um eine bevorzugte Ausführungsform. Die so erhaltenen Duroplasten werden zerkleinert und feinst vermahlen. Das so gewonnene erfindungsgemäße Pulver kann - je nach Anwendungsgebiet - bei der Herstellung von antimikrobiell wirkenden Medien diesen zugesetzt werden.

Die Polysiloxane weisen eine große wirtschaftliche Bedeutung auf. Je nach Molmasse gibt es viskose Öle, Pasten, Fette bzw. kautschukelastische bei weitmaschig- oder duroplastische - bei engmaschig vernetzten Polysyloxanen.

Besonders geeignet ist das erfindungsgemäße Pulver als Zusatz für Verfugmassen von Feuchträumen. Ein Zusatz von 0,5% Wirkstoffanteil verhindert den schwarzen Belag solcher Verfugungen, verursacht durch den Aspergillus niger-Befall.

Ein weiteres Beispiel für die Anwendung ist eine Formulierung als Schutzlack für diverse Wände, auch Spitalswände. Die Filme mit so formulierten Beschichtungen weisen eine antimikrobielle Wirkung auf.

Das erfindungsgemäße Polymergemisch kann auch als Zuschlagstoff in der Papierindustrie eingesetzt werden. Beim Produktionsprozess wird es bei der Stoffaufbereitung zugesetzt und ermöglicht die Herstellung eines antimikrobiell geschützten Papiers, was besonders für die Erzeugung von Wellpappe und Kartonagen zur Lebensmittelverpackung von Bedeutung ist. Dasselbe gilt für die Zellstoffherstellung zur Weiterverarbeitung von Binden und anderen Hygieneartikeln.

Mittels der Pulverbeschichtungstechnologie werden Metallgegenstände mit Kunststoff überzogen; bei solchen, wo antimikrobielle Eigenschaft der Oberfläche vonnöten ist, kann durch Zusatz des erfindungsgemäßen Pulvers die gewünschte Wirkung erzielt werden.

Das erfindungsgemäße Polymergemisch kann auch als antimikrobiell wirkendes Pulver allein zum Einsatz kommen, durch Bestreuen der mikrobiell anfälligen Flächen wie z.B. in Stallungen.

Das erfindungsgemäße Polymergemisch kann einen Zusatzstoff, z.B. einen Füllstoff, aufweisen.

Mit den nachfolgenden Beispielen wird die Erfindung noch näher beschrieben, wobei mit dem Beispielen 1 bis 6 die Herstellung von erfindungsgemäßen mikrobiozid wirkenden Polymergemischen in Pulverform beschrieben wird. Die Beispiele 6 bis 11 beschreiben mehrere Anwendungen des erfindungsgemäßen Pulvers.

Beispiel 1

Thermoplastisches Polyurethan (Typ Pellethane 2363-90A, Fa. DOW) wird in einen gleichläufigen Doppelschneckenextruder (Typ: Thermo Prism TSE 24 HC) in Zone 3 (von insgesamt 10 thermisch erfassbaren Zonen) eingebracht. Das Polyurethan dient als Matrix für das nachfolgend einzubringende polymere Guanidinderivat.

Als polymeres Guanidinderivat wird das Polykondensat aus 1 Mol Guanidiniumhydrochlorid und einem Gemisch aus 0,75 Mol Hexamethylendiamin und 0,25 Mol Triethylenglykolamin verwendet. Dieses Copolymer ist im Handel erhältlich ("Akacid Plus" der Fa. AKA Technology). Die Herstellung ist in der WO 06/047800 A1 beschrieben. Das Guanidinderivat wird als 75%-ige wässerige Lösung der Zone Nr.5 des Doppelschneckenextruders zudosiert. Das Guanidinderivat kann vorzugsweise-je Anwendungsbereich - in einer Menge zudosiert werden, dass sein Anteil im fertigen Gemisch mit dem Polyurethan zwischen 0, 1 % und 20% beträgt.

Die Temperaturverteilung in den einzelnen Zonen des Extruders ist wie folgt:

Zone 1 : 40 °C

Zone 2 : 170°C

Zone 3 : 210°C

Zone 4 : 210 °C

Zone 5 : 190 ° C

Zone 6 : 110 ° C

Zone7:110°C

Zone 8: 190 °C

Zone 9 : 190 ° C

Zone 10 : 180 °C

Düse: 180° C

Durch die Extrusion werden das Polyurethan und das polymere Guanidinderivat innig miteinander vermischt. Das extrudierte Gemisch wird durch ein Wasserbad gezogen und anschließend granuliert.

Das Granulat wird feinst vermahlen, vorzugsweise mit einer Mahlfeinheit von 100-300 $\mu$m. Die Vermahlung kann in kryogenen Feinmühlen erfolgen.

Beispiel 2

Ein Gemisch aus granulatförmigem Hochdruck-Polyäthylen (Typ: Borstar MB6561, Fa.Borealis), welches als Matrix dient, und pulverförmigem Poly-(hexamethylendiaminguanidiniumchlorid), dem "Akacid Forte" der Firma AKA Technology, wird in einen gleichläufigen Doppelschneckenextruder (Typ: Thermo Prism TSE 24 HC) in Zone 3 (von insgesamt 10 thermisch erfassbaren Zonen) eingebracht. Die Temperaturverteilung in den einzelnen Zonen ist wie folgt:

Zone 1 : 40 ° C

Zone 2 : 150 ° C

Zone 3 : 180°C

Zone 4 : 180°C

Zone 5 : 180 ° C

Zone 6 : 160 °C

Zone 7 : 160 °C

Zone 8 : 190 °C

Zone 9 : 190 °C

Zone 10 : 190 ° C

Düse: 170°C

Das Gemisch wird extrudiert, durch ein Wasserbad gezogen und anschließend granuliert. Dieses Granulat wird analog zu Beispiel 1 zu einem Pulver weiterverarbeitet.

Beispiel 3

Als Matrix wird Polyamid 6 ( Typ: Fürkamid B,SK1, Fa.Solvadis Polym.) in einen gleichläufigen Doppelschnek-kenextruder ( Typ: Thermo Prism TSE 24 HC) in Zone 3 (von insgesamt 10 thermisch erfassbaren Zonen) eingebracht. In der Zone Nr.5 wird das in Beispiel 1 angegebene Guanidincopolymer "Aka plus" der Fa. AKA Technology im Verhältnis 40:60 zugesetzt.
Die Temperaturverteilung in den einzelnen Zonen ist wie folgt:

Zone 1 : 40 °C

Zone 2 : 220 °C

Zone 3 : 240 °C

Zone 4 : 250 °C

Zone 5 : 230 °C

Zone 6 : 140 °C

Zone 7 : 140 °C

Zone 8 : 240 °C

Zone 9 : 240 ° C

Zone 10 : 230 ° C

Düse: 230° C

Das Gemisch wird extrudiert, durch ein Wasserbad gezogen und anschließend granuliert. Dieses Granulat wird analog zu Beispiel 1 zu einem Pulver weiterverarbeitet.

Beispiel 4

Als Matrix wird Polypropylen ( Typ: PPH7062, Fa.Total Chemicals) in einen gleichläufigen Doppelschneckenextruder ( Typ: Thermo Prism TSE 24 HC) in Zone 3 ( von insgesamt 10 thermisch erfassbaren Zonen) eingebracht. In der Zone Nr.5 wird eine 75%ige wässerige Lösung des in Beispiel 1 angegebenen Guanidincopolymers "Akacid Plus" der Fa. AKA Technology zudosiert.
Die Temperaturverteilung in den einzelnen Zonen ist wie folgt:

Zone 1 : 40 ° C

Zone 2 : 150 °C

Zone 150 ° C

Zone 4 : 160 °C

Zone 5 : 160 °C

Zone 6 : 160 ° C

Zone 7 : 160 °C

Zone 8 : 170 ° C

Zone 9 : 180 °C

Zone 10 : 180 °C

Düse: 170°C

Das Gemisch wird extrudiert, durch ein Wasserbad gezogen und anschließend granuliert. Dieses Granulat wird analog zu Beispiel 1 zu einem Pulver weiterverarbeitet.

Beispiel 5

Als Matrix wird Polycarbonat ( Typ: MakrolonRX1805, Fa.Bayer ) in einen gleichläufigen Doppelschneckenextruder ( Typ: Thermo Prism TSE 24 HC) in Zone 3 ( von insgesamt 10 thermisch erfassbaren Zonen) eingebracht. In der Zone Nr.5 wird eine 75%ige wässerige Lösung des in Beispiel 1 angegebenen Guanidincopolymers, "Akacid Plus" der Fa. AKA Technology, zudosiert. Die Wirkstoffkonzentration beträgt in diesem Fall 5%.
Die Temperaturverteilung in den einzelnen Zonen ist wie folgt:

Zone 1 : 40 ° C
Zone 2 : 150 ° C
Zone 3 : 240 °C
Zone 4 : 250 °C
Zone 5 : 230 °C
Zone 6 : 140 °C
Zone 7 : 140 °C
Zone 8 : 240 °C
Zone 9 : 240 °C
Zone 10 : 230 °C
Düse: 230° C

Das Gemisch wird extrudiert, durch ein Wasserbad gezogen und anschließend granuliert. Dieses Granulat wird analog zu Beispiel 1 zu einem Pulver weiterverarbeitet.

Beispiel 6

Als Matrix wird Acryl-Butadien-Styrol (Typ: PA727, ein thermoplastisches ABS der Fa.Chi-Mei,Taiwan) in einen gleichläufigen Doppelschneckenextruder ( Typ: Thermo Prism TSE 24 HC) in Zone 3 (von insgesamt 10 thermisch erfassbaren Zonen) eingebracht. In der Zone Nr.5 wird eine 75%ige wässerige Lösung des in Beispiel 1 angegebenen Guanidincopolymers "Akacid Plus" der Fa. AKA Technology zudosiert.
Die Temperaturverteilung in den einzelnen Zonen ist wie folgt:

Zone 1 : 40 °C
Zone 2 : 170 ° C
Zone 3 : 200 °C
Zone 4 : 200 °C
Zone 5 : 180 ° C
Zone 6 : 110 °C
Zone 7 : 110°C
Zone 8 : 190 ° C
Zone 9 : 190 °C
Zone 10 : 180 °C

Düse: 180°C

Das Gemisch wird extrudiert, durch ein Wasserbad gezogen und anschließend granuliert. Dieses Granulat wird analog zu Beispiel 1 zu einem Pulver weiterverarbeitet.

Beispiel 7: Dispersionsfarbe

Material

Durovit Acryl-Innendispersionsfarbe ist ein handelsüblicher wasserverdünnbarer Acryldispersionslack mit <2,5w% N,N-Dimethylformamid der Firma Knuchel Farben AG, Steinackerweg 11, CH-4537 Wiedlisbach.

In die Dispersionsfarbe wurde das erfindungsgemäße Pulver auf der Basis einer Acryl-Butadien-Styrol Matrix (ABS) gemäß Beispiel 6 mit 0%, 0,2%, 0,4% und 0,8% Wirkstoff gleichmäßig eingerührt. Anschließend wurden die dickflüssigen Farbmischungen mit einem Pinsel auf jeweils vier Metallplättchen (5cm x 5cm) je Konzentration in einer gleichmäßigen Schicht aufgetragen. Diese Beschichtungen wurden 24 Stunden bei 22°C zur Austrocknung gelagert. Die fungizide Wirksamkeit der trockenen Farboberflächen wurde mit den Testkeimen *Aspergillus niger* DSM 1957und *Penicillium funiculosum* DSM 1944 unter Anwendung der Standardmethode der "American Society for Testing and Materials"ASTM D 5590 (2005) "Determining the resistance of paint films and related coatings to fungal defacement by accelerated four-week agar plate assay" durchgeführt. Je stärker die Pilze wachsen, desto schwächer ist die Wirksamkeit des zu testenden Materials. Zur Evaluierung werden die Farbproben auf ein in Petrischalen (Durchmesser 90mm) befindliches Kartoffeldextrose-Nährmedium gelegt. Medium und Proben werden dann mit einer Sporenlösung der beiden Testkeime beimpft.

Dazu werden die Pilze auf einem Malzextraktagar-Nährboden in Petrischalen (Durchmesser 90mm) bei 24°C im Dunkeln angezogen. Nach zwei Wochen werden von den gut wachsenden und sporulierenden Pilzkulturen wässrige Sporenlösungen hergestellt und mittels eines Hämocytometer auf eine Sporenkonzentration von jeweils $10^4$ Sporen/mL eingestellt. Die beiden Sporenlösungen werden 1:1 vermischt. und auf die Probeflächen und die nicht bedeckten Flächen des Nährmediums mit Hilfe eines Handsprühers (30mL) so verteilt, das sich auf den Oberflächen feine Tröpfchen bilden.

Die visuelle Bewertung des Pilzwachstums erfolgt einen Monat lang in wöchentlichen Abständen nach folgender Skala:

0 = Kein Pilzwachstum auf den Platten
1 = <10% der Platte mit Pilzen bewachsen (Spuren)
2 = 10-30% der Platte mit Pilzen bewachsen (wenig Wachstum)
3 = > 30-60% der Platte mit Pilzen bewachsen (mittleres Wachstum)
4 = > 60-100% der Platte mit Pilzen bewachsen (starkes Wachstum)

Treten Werte von gleich oder kleiner als 1 auf, so wird die Prüfsubstanz als fungistatisch angesehen.

Ergebnisse

Die Ergebnisse der fungiziden Wirksamkeit der Farboberflächen sind in Tabelle 1 dargestellt. Daraus lässt sich entnehmen, dass das Pilzwachstum auf den Probeflächen bei einer Wirkstoffkonzentration von 0% und 0,2% ab der zweiten Woche nach der Inokulation begann. Das nicht mit Probeplatten bedeckte Kartoffeldextrosemedium war bereits nach Ablauf der ersten Woche vollständig mit Pilzmyzel überwachsen. Bei 0,4% Wirkstoffgehalt setzte das Pilzwachstum erst eine Woche später ein und bei 0,8% war kein Pilzwachstum mehr zu beobachten.

Die Wirkung des Wirkstoffes ist konzentrationabhängig. Bei 0,2% wächst das Pilzgemisch ähnlich stark wie in der Kontrolle mit 0%. Bei einem Zusatz von 0,4% werden nur noch Werte von 0,3 erreicht und bei 0,8% wird kein Pilzwachstum mehr beobachtet. Abhängig von der Konzentration des Wirkstoffes wird das Pilzwachstum verzögert oder sogar verhindert.

Tabelle 1: Bestimmung der fungiziden Wirkung von Durovit Innendispersionsfarbe mit oder ohne Pulverzusatz auf Metallplatten (5x5cm) gegen *Aspergillus niger* und *Penicillium funiculosum* nach ASTM international: D 5590 (2005), n = 4. Gesamtdauer des Versuches = 4 Wochen (09.07.-04.08.2009).

| Farbkodierung | Boniturwert | Boniturwert | Boniturwert | Boniturwert |
|---|---|---|---|---|
| | Woche 1 | Woche 2 | Woche 3 | Woche 4 |
| Durovit 0 | 0 | 1 | 1 | 1 |

(fortgesetzt)

| Farbkodierung | Boniturwert | Boniturwert | Boniturwert | Boniturwert |
|---|---|---|---|---|
| | Woche 1 | Woche 2 | Woche 3 | Woche 4 |
| Durovit 0,2% | 0 | 1 | 1 | 1 |
| Durovit 0,4% | 0 | 0 | 0,3 | 0,3 |
| Durovit 0,8% | 0 | 0 | 0 | 0 |

Beispiel 8: Dispersionsfarbe

Material

Sax Walith Power Acryl-Innendispersionsfarbe ist ein handelsüblicher wasserverdünnbarer Acryldispersionslack der Firma Sax Farben AG, CH Urdorf.

In die Dispersionsfarbe wurde das erfindungsgemäße Pulver auf der Basis eines Thermoplast.PU (Typ: Pellethane 2363-90A, Fa.DOW) gemäß Beispiel 1 mit 0%, 0,5%, 0,8% und 1,0% Wirkstoff gleichmäßig eingerührt. Anschließend wurden die dickflüssigen Farbmischungen mit einem Pinsel auf jeweils vier Metallplättchen (5cm x 5cm) je Konzentration in einer gleichmäßigen Schicht aufgetragen. Diese Beschichtungen wurden 24 Stunden bei 22°C zur Austrocknung gelagert. Die fungizide Wirksamkeit der trockenen Farboberflächen wurde mit den Testkeimen *Aspergillus niger* DSM 1957und *Penicillium funiculosum* DSM 1944 unter Anwendung der Standardmethode der "American Society for Testing and Materials"ASTM D 5590 (2005) "Determining the resistance of paint films and related coatings to fungal defacement by accelerated four-week agar plate assay" durchgeführt. Je stärker die Pilze wachsen, desto schwächer ist die Wirksamkeit des zu testenden Materials. Zur Evaluierung werden die Farbproben auf ein in Petrischalen (Durchmesser 90mm) befindliches Kartoffeldextrose-Nährmedium gelegt. Medium und Proben werden dann mit einer Sporenlösung der beiden Testkeime beimpft.

Dazu werden die Pilze auf einem Malzextraktagar-Nährboden in Petrischalen (Durchmesser 90mm) bei 24°C im Dunkeln angezogen. Nach zwei Wochen werden von den gut wachsenden und sporulierenden Pilzkulturen wässrige Sporenlösungen hergestellt und mittels eines Hämocytometer auf eine Sporenkonzentration von jeweils $10^4$ Sporen/mL eingestellt. Die beiden Sporenlösungen werden 1:1 1 vermischt. und auf die Probeflächen und die nicht bedeckten Flächen des Nährmediums mit Hilfe eines Handsprühers (30mL) so verteilt, dass sich auf den Oberflächen feine Tröpfchen bilden.

Die visuelle Bewertung des Pilzwachstums erfolgt einen Monat lang in wöchentlichen Abständen nach folgender Skala:

0 = Kein Pilzwachstum auf den Platten
1 = <10% der Platte mit Pilzen bewachsen (Spuren)
2 = 10-30% der Platte mit Pilzen bewachsen (wenig Wachstum)
3 = > 30-60% der Platte mit Pilzen bewachsen (mittleres Wachstum)
4 = > 60-100% der Platte mit Pilzen bewachsen (starkes Wachstum)

Treten Werte von gleich oder kleiner als 1 auf, so wird die Prüfsubstanz als fungistatisch angesehen.

Ergebnisse

Die Ergebnisse der fungiziden Wirksamkeit der Farboberflächen sind in Tabelle 2 dargestellt. Daraus lässt sich entnehmen, dass das Pilzwachstum auf den Probeflächen bei einer Wirkstoffkonzentration von 0% und 0,5% ab der ersten Woche nach der Inokulation begann. Das nicht mit Probeplatten bedeckte Kartoffeldextrosemedium war zu diesem Zeitpunkt vollständig mit Pilzmyzel überwachsen. Bei 0,8% und bei 1,0% Wirkstoffgehalt war kein Pilzwachstum mehr zu beobachten.

Die Wirkung des Wirkstoffes ist konzentrationabhängig. Bei 0,5% wächst das Pilzgemisch ähnlich stark wie in der Kontrolle mit 0%. Bei einem Zusatz von 0,8% und bei 1,0% wird kein Pilzwachstum mehr beobachtet. Abhängig von der Konzentration des Wirkstoffes wird das Pilzwachstum verzögert (0,5%) oder sogar verhindert (0,8% und 1,0%).

Tabelle 2: Bestimmung der fungiziden Wirkung von Sax Walith Power Innendispersionsfarbe mit oder ohne Pulverzusatz auf Metallplatten (5x5cm) gegen *Aspergillus niger* und *Penicillium funiculosum* nach ASTM international: D 5590 (2005), n = 4. Gesamtdauer des Versuches = 4 Wochen.

| Farbkomposition | Boniturwert | | | |
|---|---|---|---|---|
| | Woche 1 | Woche 2 | Woche 3 | Woche 4 |
| Sax Walith Power 0% Wirkstoff | 1,8 | 2 | 2 | 2 |
| Sax Walith Power + 0,5% Wirkstoff | 1 | 2 | 2 | 2 |
| Sax Walith Power + 0,8% Wirkstoff | 0 | 0 | 0 | 0 |
| Sax Walith Power + 1 % Wirkstoff | 0 | 0 | 0 | 0 |

Beispiel 9: Zwei-Komponenten-Lack

Material

Bei Reaktivsystemen, welche aus zwei oder mehreren Komponenten bestehen, wird das erfindungsgemäße Pulver entweder dem Harz oder dem Härter zugesetzt. Bei den Beschichtungsmaterialien handelt es sich um Epoxide, Polyurethane, Silikone oder Polyester.

Harz und Härter des handelsüblichen Zwei-Komponenten-Produktes "Eposilan Schutzlack 2K", wurde von der Fa. Scheidel Innovative Chemie, Hirschaid, Deutschland bezogen. Dieser Speziallack enthält Silikonharz und Epoxidharz auf der Basis von Epoxy-Silan. Harz und Härter werden im Verhältnis 5:1 Volumenanteile angerührt bis eine gleichmäßige homogene Masse entsteht und so weiter verarbeitet.

In den Härter wurde zuerst das erfindungsgemäße Pulver auf der Basis eines Polyamid 6 (Typ: Fürkamid B, SK1, Fa.Solvadis Polym.) gemäß Beispiel 3 mit 0%, 0,25%, 0,5% und 1,0% Wirkstoff und in einem weiteren Versuch (Beispiel 10) mit 0,4% Wirkstoff gleichmäßig eingerührt. Anschließend wurde das Harz im Verhältnis 5:1 Volumenanteilen mit dem so präparierten Härter vermischt. Diese dickflüssigen Farbmischungen wurden mit einem Pinsel auf jeweils vier Metallplättchen (5cm x 5cm) je Konzentration in einer gleichmäßigen Schicht aufgetragen. Diese Beschichtungen wurden 24 Stunden bei 22°C zur Austrocknung gelagert.

Zur quantitativen Untersuchung der bakteriziden Wirkung des erfindungsgemäßen Pulvers in den trockenen Lackoberflächen wurden die Proben mit den Testkeimen *Escherichia coli* (ATCC 10536) oder *Staphylococcus aureus* (ATCC 6538). gemäß des Testverfahrens des Japanischen Industrie Standards JIS Z 2801 (2000) inokuliert. Die Testflächen wurden mit 0,2 mL einer Keimsuspension entsprechender Keimdichte ($10^5$ pro mL beimpft) und mit PE-Folie abgedeckt. Die Inkubation erfolgte bei einer Luftfeuchtigkeit > 90% und einer Temperatur von 35°C über 24 Stunden. Danach wurden die Bakterien mit physiologischer Kochsalzlösung von den Testflächen abgewaschen und die Lebendkeimzahlen nach 24 oder 48 Stunden ermittelt. Die Berechnung der Reduktionszahlen (R-Werte) erfolgte nach folgender Formel:

$$R = [\log (B/A) - \log (C/A)]$$

R-Wert: Wert der antimikrobiellen Aktivität, Reduktionszahl
A-Wert: Lebendkeimzahl am wirkstofffreien Vergleichsmuster direkt nach dem Beimpfen
B-Wert: Lebendkeimzahl am wirkstofffreien Vergleichsmuster nach 24h Inkubation
C-Wert: Lebendkeimzahl am wirkstoffhaltigen Teststück nach 24-48h Inkubation

Als antimikrobiell aktiv werden alle die Stoffe angesehen, die zu einer Log-Reduktion von zwei oder mehr führen.

Ergebnisse

Die Ergebnisse der bakteriziden Wirksamkeit der Farboberflächen sind in der Tabelle 3a dargestellt. Das erfindungsgemäße Pulver im Lack unterdrückte signifikant das Bakterienwachstum. Die Wirkung des Probematerials war abhängig von der Wirkstoffkonzentration. Mit dem Zusatz von 0,25% Wirkstoff wurde die Zahl der Bakterien von *E. coli* als auch von S. *aureus* zwischen einer und zwei Log-Stufen vermindert, was eine Reduktion von rund 91% bzw. 93% ausmacht. Mit dem Zusatz von 0,5% Wirkstoff war die Log-Reduktion für beide Bakterien größer als 2, was mehr als 96% für *E. coli* ausmachte und fast 100% für S. *aureus.* Bei einem Wirkstoffgehalt von 1,0% war die Log-Reduktion für beide Bakterienstämme größer als 3, was eine Verminderung der Bakterienzahlen von fast 100% bedeutet.

Tabelle 3a: Bakterizide Wirkung des erfindungsgemäßen Pulvers eingearbeitet in Eposilan 2K Klarlack auf Metallplatten (5cm x 5cm) gegen *E. coli* and *S. aureus.*

| Probe | Mittelwert | Reduktion | Log-Reduktion | |
|---|---|---|---|---|
| | (n=5) | (%) | Mean | sd |
| *E. coli* | | | | |
| EPS 0% | 2,19E+06 | 0,00 | | |
| EPS 0,25% | 1,95E+05 | 91,11 | 1,0 | 0,1 |
| EPS 0,5% | 9,62E+04 | 95,62 | >2 | 1,2 |
| EPS 1,0% | 1,77E+03 | 99,92 | >3,2 | 0,6 |
| | | | | |
| *S. aureus* | (n=2) | | | |
| EPS 0% | 9,77E+05 | 0,00 | | |
| EPS 0,25% | 7,02E+04 | 92,81 | 1,6 | 0,7 |
| EPS 0,5% | 2,34E+03 | 99,76 | 2,2 | 1,2 |
| EPS 1,0% | 2,75E+02 | 99,97 | >3,2 | 0,6 |

Tabelle 3b : Bakterizide Wirkung von des erfindungsgemäßen Pulvers eingearbeitet in Eposilan 2K Klarlack auf Metallplatten (5cm x 5cm) gegen *E. coli* und *S. aureus.*

| Wiederholung 1 | E. coli |
|---|---|
| A-Wert [KBE/Probe] | 3,20E+05 |
| B-Wert [KBE/Probe] | 5,32E+05 |
| C-Wert [KBE/Probe] 0,4% | 1,91 E+04 |
| R-Wert | 1,4 |
| | |
| Wiederholung 2 | E. coli |
| A-Wert [KBE/Probe] | 1,49E+06 |
| B-Wert [KBE/Probe] | 5,31E+05 |
| C-Wert [KBE/Probe] 0,4 % | 1,97E+04 |
| R-Wert | 1,4 |

| Wiederholung 1 | S. aureus |
|---|---|
| A-Wert [KBE/Probe] | 3,80E+05 |
| B-Wert [KBE/Probe] | 3,90E+06 |
| C-Wert [KBE/Probe] 0,4% | 3,00E+02 |
| R-Wert | 4,1 |

(fortgesetzt)

| Wiederholung 1 | S. aureus |
|---|---|
|  |  |
| Wiederholung 2 | S. aureus |
| EPOSILAN |  |
| A-Wert [KBE/Probe] | 8.35E+05 |
| B-Wert [KBE/Probe] | 6,80E+06 |
| C-Wert [KBE/Probe] 0,4% | 9,90E+01 |
| R-Wert | 4,8 |

Beispiel 10: Zwei-Komponenten-Lack

Material

Bei Reaktivsystemen, welche aus zwei oder mehreren Komponenten bestehen, wird das erfindungsgemäße Pulver entweder dem Harz oder dem Härter zugesetzt. Bei den Beschichtungsmaterialien handelt es sich um Epoxide, Polyurethane, Silikone oder Polyester.

Harz und Härter des handelsüblichen Zwei-Komponenten-Produktes "Eposilan Schutzlack 2K" , wurde von der Fa. Scheidel Innovative Chemie, Hirschaid, Deutschland bezogen. Dieser Speziallack enthält Silikonharz und Epoxidharz auf der Basis von Epoxy-Silan. Harz und Härter werden im Verhältnis 5:1 Volumenanteile angerührt bis eine gleichmäßige homogene Masse entsteht und so weiter verarbeitet.

In den Härter wurde das erfindungsgemäße Pulver auf der Basis eines Polyamid 6 (Typ:

Fürkamid B,SK1, Fa.Solvadis Polym.) gemäß Beispiel 3 mit 0,4% Wirkstoff gleichmäßig eingerührt. Anschließend wurde das Harz im Verhältnis 5:1 Volumenanteilen mit dem so präparierten Härter vermischt. Diese dickflüssigen Farbmischungen wurden mit einem Pinsel auf jeweils vier Metallplättchen (5cm x 5cm) je Konzentration in einer gleichmäßigen Schicht aufgetragen. Diese Beschichtungen wurden 24 Stunden bei 22°C zur Austrocknung gelagert.

Zur quantitativen Untersuchung der bakteriziden Wirkung des erfindungsgemäßen Pulvers in den trockenen Lackoberflächen wurden die Proben mit den Testkeimen *Escherichia coli* (ATCC 10536) oder *Staphylococcus aureus* (ATCC 6538). gemäß des Testverfahrens des Japanischen Industrie Standards JIS Z 2801 (2000) inokuliert. Die Testflächen wurden mit 0,2 mL einer Keimsuspension entsprechender Keimdichte ($10^5$ pro mL beimpft) und mit PE-Folie abgedeckt. Die Inkubation erfolgte bei einer Luftfeuchtigkeit > 90% und einer Temperatur von 35°C über 24 Stunden. Danach wurden die Bakterien mit physiologischer Kochsalzlösung von den Testflächen abgewaschen und die Lebendkeimzahlen nach 24 oder 48 Stunden ermittelt. Die Berechnung der Reduktionszahlen (R-Werte) erfolgte nach folgender Formel:

$$R = [\log (B/A) - \log (C/A)]$$

R-Wert: Wert der antimikrobiellen Aktivität, Reduktionszahl
A-Wert: Lebendkeimzahl am wirkstofffreien Vergleichsmuster direkt nach dem Beimpfen
B-Wert: Lebendkeimzahl am wirkstofffreien Vergleichsmuster nach 24h Inkubation
C-Wert: Lebendkeimzahl am wirkstoffhaltigen Teststück nach 24-48h Inkubation

Als antimikrobiell aktiv werden alle die Stoffe angesehen, die zu einer Log-Reduktion von zwei oder mehr führen.

Ergebnisse

Die Ergebnisse der bakteriziden Wirksamkeit der Farboberflächen sind in Tabelle 3c dargestellt. Das erfindungsgemäße Pulver im Lack unterdrückte signifikant das Bakterienwachstum. Mit dem Zusatz von 0,4% Wirkstoff wurde die Zahl der Bakterien von *E. coli* um 1,4 und S. *aureus* zwischen 4 und 5 Log-Stufen vermindert.

Tabelle 3c : Bakterizide Wirkung von des erfindungsgemäßen Pulvers eingearbeitet in Eposilan 2K Klarlack auf Metallplatten (5cm x 5cm) gegen *E. coli* und S. *aureus.*

| Wiederholung 1 | E. coli |
|---|---|
| A-Wert [KBE/Probe] | 3,20E+05 |
| B-Wert [KBE/Probe] | 5,32E+05 |
| C-Wert [KBE/Probe] 0,4% | 1,91 E+04 |
| R-Wert | 1,4 |
| Wiederholung 2 | E. coli |
| A-Wert [KBE/Probe] | 1,49E+06 |
| B-Wert [KBE/Probe] | 5,31E+05 |
| C-Wert [KBE/Probe] 0,4 % | 1,97E+04 |
| R-Wert | 1,4 |

| Wiederholung 1 | S. aureus |
|---|---|
| A-Wert [KBE/Probe] | 3,80E+05 |
| B-Wert [KBE/Probe] | 3,90E+06 |
| C-Wert [KBE/Probe] 0,4% | 3,00E+02 |
| R-Wert | 4,1 |
| | |
| Wiederholung 2 | S. aureus |
| EPOSILAN | |
| A-Wert [KBE/Probe] | 8,35E+05 |
| B-Wert [KBE/Probe] | 6,80E+06 |
| C-Wert [KBE/Probe] 0,4% | 9,90E+01 |
| R-Wert | 4,8 |

**Patentansprüche**

1. Mikrobiozid wirkendes Polymergemisch, welches ein erstes Polymer als Matrix aufweist, in welche ein zweites Polymer eingebracht ist, wobei das zweite Polymer ein polymeres Guanidin- oder Biguanidinderivat ist, **dadurch gekennzeichnet, dass** das mikrobiozid wirkende Polymergemisch als Pulver ausgebildet ist.

2. Polymergemisch nach Anspruch 1, **dadurch gekennzeichnet, dass** die mittlere Teilchengröße des Pulvers im Bereich von 10 nm und 1.000 $\mu$m, insbesondere im Bereich von 50 $\mu$m und 100 $\mu$m, liegt.

3. Polymergemisch nach einem der Ansprüche 1 oder 2, **dadurch gekennzeichnet, dass** das polymere Biguanidinderivat ein Poly(hexamethylen)biguanid ist.

4. Polymergemisch nach einem der Ansprüche 1 oder 2, **dadurch gekennzeichnet, dass** das polymere Guanidinderivat auf Basis eines Alkylendiamins und/oder eines Oxyalkylendiamins ist.

5. Polymergemisch nach Ansprüche 4, **dadurch gekennzeichnet, dass** ein polymeres Guanidinderivat vorgesehen ist, welches das Alkylendiamin und das Oxyalkylendiamin im Molverhältnis zwischen 4:1 und 1:4 enthält.

6. Polymergemisch nach einem der Ansprüche 4 oder 5, **dadurch gekennzeichnet, daß** die Aminogruppen des Alkylendiamins und/oder des Oxyalkylendiamins endständig sind.

7. Polymergemisch nach einem der Ansprüche 4 bis 6, **dadurch gekennzeichnet, daß** zur Herstellung des polymeren Guanidinderivates als Alkylendiamin eine Verbindung der allgemeinen Formel

$$NH_2(CH_2)_nNH_2$$

vorgesehen ist, in welcher n eine ganze Zahl zwischen 2 und 10, insbesondere 6, ist.

8. Polymergemisch nach einem der Ansprüche 4 bis 7, **dadurch gekennzeichnet, daß** zur Herstellung des polymeren Guanidinderivates als Oxyalkylendiamin eine Verbindung der allgemeinen Formel

$$NH_2[(CH_2)_2O)]_n(CH_2)_2NH_2$$

vorgesehen ist, in welcher n eine ganze Zahl zwischen 2 und 5, insbesondere 2, ist.

9. Polymergemisch nach einem der Ansprüche 4 bis 8, **dadurch gekennzeichnet, dass** als polymeres Guanidinderivat ein Polyoxyalkylen-Guanidin auf Basis von Triethylenglykoldiamin (relative Molekularmasse: 148), von Polyoxypropylendiamin (relative Molekularmasse: 230) und/oder von Polyoxyethylendiamin (relative Molekularmasse: 600) vorgesehen ist.

10. Polymergemisch nach einem der Ansprüche 4 bis 9, **dadurch gekennzeichnet, dass** als das polymere Guanidinderivat Poly-[2-(2-ethoxy-ethoxyethyl)-guanidinium-hydrochlorid] mit mindestens 3 Guanidinresten vorgesehen ist.

11. Polymergemisch nach einem der Ansprüche 1 bis 10, **dadurch gekennzeichnet, dass** die mittlere Molekularmasse des polymeren Guanidinderivates im Bereich 500 bis 3.000 liegt.

12. Polymergemisch nach einem der Ansprüche 1 bis 11, **dadurch gekennzeichnet, dass** das erste Polymer ein Thermoplast, ein Duroplast oder ein Elastomer ist.

13. Polymergemisch nach einem der Ansprüche 1 bis 12, **dadurch gekennzeichnet, dass** es einen Zusatzstoff aufweist.

14. Verwendung eines Polymergemisches nach einem der Ansprüche 1 bis 13 als mikrobiozid wirkender Zusatz zu Lacken, Farben, Beschichtungsmaterialien und Fugenmasse.

15. Verwendung eines Polymergemisches nach einem der Ansprüche 1 bis 13, als Streu-Desinfektionsmittel.

EP 2 230 259 A1

Europäisches
Patentamt
European
Patent Office
Office européen
des brevets

**EUROPÄISCHER RECHERCHENBERICHT**

Nummer der Anmeldung

EP 09 45 0153

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 0 619 272 A (AGFA GEVAERT NV [BE]) 12. Oktober 1994 (1994-10-12) * Ansprüche 1,2 * * Seite 3, Zeile 43 - Zeile 44 * ----- | 1-15 | INV. C08B37/14 C08G73/02 |
| A,D | EP 0 439 698 A (DEGUSSA [DE]) 7. August 1991 (1991-08-07) * Ansprüche * ----- | 1 | |
| X | EP 1 918 306 A (UNIV NEW BRUNSWICK [CA]) 7. Mai 2008 (2008-05-07) * Ansprüche 1,5 * * Beispiele * ----- | 1-15 | |
| X | US 2007/270552 A1 (ZHENG ANNA [CN] ET AL) 22. November 2007 (2007-11-22) * Ansprüche 14-16 * ----- | 1-15 | |
| | | | RECHERCHIERTE SACHGEBIETE (IPC) C08B C08G |

Der vorliegende Recherchenbericht wurde für alle Patentansprüche erstellt

| Recherchenort | Abschlußdatum der Recherche | Prüfer |
|---|---|---|
| München | 8. Januar 2010 | West, Nuki |

**ANHANG ZUM EUROPÄISCHEN RECHERCHENBERICHT**
**ÜBER DIE EUROPÄISCHE PATENTANMELDUNG NR.**

EP 09 45 0153

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-01-2010

| Im Recherchenbericht angeführtes Patentdokument | Datum der Veröffentlichung | Mitglied(er) der Patentfamilie | Datum der Veröffentlichung |
|---|---|---|---|
| EP 0619272 A | 12-10-1994 | DE 69317259 D1 | 09-04-1998 |
| | | DE 69317259 T2 | 01-10-1998 |
| | | JP 2724435 B2 | 09-03-1998 |
| | | JP 7000975 A | 06-01-1995 |
| | | US 5423990 A | 13-06-1995 |
| EP 0439698 A | 07-08-1991 | DE 4002403 A1 | 01-08-1991 |
| EP 1918306 A | 07-05-2008 | CA 2608824 A1 | 30-04-2008 |
| | | US 2008139441 A1 | 12-06-2008 |
| US 2007270552 A1 | 22-11-2007 | AT 400596 T | 15-07-2008 |
| | | AU 2003221218 A1 | 29-09-2003 |
| | | WO 03078490 A1 | 25-09-2003 |
| | | CN 1445270 A | 01-10-2003 |
| | | DK 1486519 T3 | 17-11-2008 |
| | | EP 1486519 A1 | 15-12-2004 |
| | | ES 2311091 T3 | 01-02-2009 |
| | | JP 2005520877 T | 14-07-2005 |
| | | US 2005133952 A1 | 23-06-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0185676 A
- WO 2006047800 A

- WO 06047800 A1

**In der Beschreibung aufgeführte Nicht-Patentliteratur**

- Antibiotika Monitor. 01. Februar 2006

- Determining the resistance of paint films and related coatings to fungal defacement by accelerated four-week agar plate assay. *American Society for Testing and Materials,* 2005